# EUROPEAN PATENT SPECIFICATION

(11) **EP 2 688 629 B1**
(45) Date of publication and mention of the grant of the patent: **14.12.2022**
(21) Application number: 12713441.9
(22) Date of filing: 13.03.2012
(51) Int. Cl.: A61M 25/00, A61F 5/451

(54) **URINE COLLECTION ASSEMBLY AND METHOD OF MANUFACTURE THEREOF**
URINSAMMELANORDNUNG UND VERFAHREN ZUR HERSTELLUNG EINER SOLCHEN ANORDNUNG
ENSEMBLE ET PROCÉDÉ DE FABRICATION DE COLLECTE D'URINE

(30) Priority: 25.03.2011 US 201161467679 P
(43) Date of publication of application: 29.01.2014
(73) Proprietor: Hollister Incorporated, Libertyville, IL 60048 (US)
(72) Inventor: O'BRIEN, Daniel, Calry, County Sligo (IE); CREAVEN, Martin, P., Ballina, County Mayo (IE); MURRAY, Michael, Ballina, County Mayo (IE); JENSEN, Bent, DK-4652 Haarlev (DK); Hannon David, Ballina, Co. Mayo (IE)
(74) Representative: FRKelly
(86) International application number: PCT/US2012/028862
(87) International publication number: WO 2012/134804

(56) References cited:
- EP-A1- 2 042 211
- WO-A2-2007/050685
- WO-A2-2007/106431

## Description

### Field of the Disclosure

The present disclosure relates generally to urine collection assemblies and, more particularly, to a closed urine collection assembly and method of manufacturing a closed urine collection assembly utilizing a conventional catheter having a tapered funnel.

### Background of the Disclosure

Intermittent urinary catheterization is a good option for many users who suffer from various abnormalities of the urinary system. A commonly encountered situation is where single use, individually packaged, sterile catheters are used. An important criterion for any single use product is the cost of the product, i.e., a less expensive product is desired and valued.

It is also common for catheters to be provided with a surface treatment using a lubricant to reduce friction in order to allow for easier and less traumatic insertion of the catheter. Currently, there are two major categories of catheters for the drainage of urine from the bladder which have lubricated surfaces, i.e., gel lubricated catheters and hydrophilic coated catheters.

The gel lubricated catheters are made easier to insert by application to the catheter surface of a water-based gel that can be applied by the user or health care provider, or more conveniently, it can be supplied with the packaged catheter. Typically, a system is provided with the packaged catheter to apply the gel to the surface of the catheter. This system may be one where the gel is placed on the catheter surface before or during the catheter packaging operation or one where the gel is applied to the catheter surface as the catheter is inserted by the user or health care provider.

In a hydrophilic coated catheter, the catheter is provided with a thin hydrophilic coating which is adhered to the outer surface of the catheter. When this coating is activated by swelling in contact with a hydrating agent such as water, it provides a surface having a lubricious and extremely low coefficient of friction. In earlier versions, a sterile, individually packaged single use catheter is provided in a dry state, and the user or health care provider opens the package and pours water into the package to hydrate the coating. After doing so, the user or health care provider would wait 30 seconds and remove the catheter from the package ready for insertion.

A more recent version of the hydrophilic coated catheter is where the catheter is provided in a package that already contains enough loose liquid water to cause it to be fully immersed. For this product, the user simply opens the package and removes the catheter ready for insertion without the need to add water and wait 30 seconds. Other products provide the amount of liquid water necessary for immersion of the catheter, e.g., in a separate compartment of the package. With these products, the separate compartment is opened to allow the liquid immersion water to enter the catheter-containing chamber for direct contact with the hydrophilic coated surface. Depending on the product, and on the amount of water in the separate chamber, the user may be asked to manipulate the package to bathe the catheter surface in the hydrating liquid in order to activate the hydrophilic coating on the catheter surface before the catheter is removed from the package ready for insertion by the user or health care provider.

Still more recently, the need for liquid immersion of a catheter having a hydrophilic coating or surface has been eliminated. In commonly owned U.S. Patent Nos. 7,380,658, it is taught to use a vapor atmosphere within a package containing such a catheter in order to hydrate the hydrophilic coating or surface of the catheter. In some instances, it is desirable to have an entire urine collection assembly which includes both a catheter and a urine collection bag. Such a urine collection assembly makes it possible to have an entirely closed system wherein, following insertion of the catheter through the urethra and into the bladder, urine can be drained from the bladder through the catheter and directly into the urine collection bag. Following use of the urine collection assembly, the catheter can be withdrawn and the assembly can be discarded, e.g., after draining urine from the bag.

As will be appreciated, the provision of an entire urine collection assembly necessarily increases the overall cost. Since for most users it is necessary to undergo catheterization frequently, cost is an important factor to be taken into consideration. As a result, any possible reduction of cost of a urine collection assembly makes its use more desirable to more users.

In addition to cost, there are other factors that are important in providing a useful and desirable urine collection assembly. For instance, it would be desirable for such an assembly to comprise a closed system which thereby substantially eliminates any possibility of leakage at the juncture of the catheter and the collection bag. Also, it would be desirable to utilize a hydrophilic coating or surface on the catheter due to the highly lubricious nature of such coatings or surfaces and the ability to eliminate use of messy gels. Further, it would be desirable to avoid utilization of solvents or adhesives in the manufacture of a urine collection assembly in order to ensure potentially dangerous solvent or adhesive vapors are not present in the urine collection bag. Additionally, it would be desirable for the urine collection bag to include an anti-reflux feature and for the entire urine collection assembly to be such as to permit relatively compact packaging.

WO2008103644 discloses a device for connecting a catheter assembly to a collection receptacle.

The device is situated at a distal portion of the catheter assembly and the connection made may be either temporary or permanent using snap-fit, pressure-fit or other similar mechanism. US2008172042 discloses a urinary catheter assembly having a catheter, a sheath enclosing an insertable portion of the catheter, and a free end partially open to allow fluid communication between the lumen of the sheath and an environment outside the sheath. WO 2007/050685 A2 discloses a pre-wetted intermittent catheter apparatus.

The aforementioned goals are realised by the method of manufacturing a packaged ready-to-use urine collection assembly according to claim 1.

### Summary of the Disclosure

Accordingly, the disclosure is directed, according to an example not forming part of the invention, to a urine collection assembly comprising a catheter and a urine collection bag. The catheter has a proximal insertion end and a distal end having a portion of a tapered funnel integrally associated therewith. The portion of the tapered funnel associated with the distal end of the catheter comprises only a cylindrical barrel portion.

The urine collection bag has a cylindrical attachment port and the cylindrical barrel portion is sealed in the cylindrical attachment port. In this manner, urine being drained from the bladder by the catheter can pass directly into the urine collection bag through a sealed joint between the catheter and the urine collection bag. Preferably, the urine collection assembly including the catheter and urine collection bag is provided in a packaged, ready-to- use condition.

In an exemplary embodiment, the urine collection bag is formed of a flexible sheet material and the attachment port is sealed into the periphery of the urine collection bag. The flexible sheet material can advantageously comprise two generally rectangular sheets of EVA/polyethylene film sealed substantially entirely about the peripheral edges thereof except where the attachment port is located as will be explained in greater detail below. Further, the cylindrical attachment port is preferably a co-extruded tube which is formed to have an EVA outer surface and a PVC inner surface.

With the cylindrical attachment port formed in this manner, the cylindrical barrel portion which is also conventionally formed of PVC is preferably sealed to the inner surface of the co-extruded tube by spin welding.

In an exemplary embodiment, the catheter is disposed externally of the urine collection bag and includes a hydrated hydrophilic outer surface. Advantageously, the catheter can also include an introducer at or near the proximal insertion end of the catheter and a sleeve substantially covering the catheter. Still further, the urine collection assembly preferably includes an anti-reflux valve sealed to the cylindrical attachment port within the urine collection bag.

The invention is directed to a method of manufacturing a packaged ready-to-use urine collection assembly according to claim 1.

Preferably, the method includes the steps of forming the urine collection bag of a flexible sheet material and sealing the attachment port into the sheet material. It may also advantageously include the step of using two sheets of EVA/polyethylene film for the flexible sheet material and sealing the two sheets about the peripheral edges thereof. It may also preferably include the step of providing a co-extruded tube having an EVA outer surface and a PVC inner surface for the cylindrical attachment port. In addition, the method may advantageously include the cylindrical barrel portion being formed of PVC and the sealing step being performed by spin welding. The spin welding step is preferably performed by holding the catheter in a stationary position and spinning the urine collection bag relative to the catheter. The spin welding step may advantageously include spinning the urine collection bag relative to the catheter at a rotational speed of between 1900 and 3000 RPMs. Also, the spin welding step preferably includes spinning the urine collection bag relative to the catheter for between 14 and 16 revolutions.

Preferably, the method further includes the step of providing the urine collection bag in a generally rectangular shape, folding the urine collection bag at least once generally perpendicular to the direction of the catheter, performing the spin welding step, folding the urine collection bag at least once in the direction of the catheter, and banding the urine collection bag in a folded condition.

As for other aspects of the method, it can include the step of providing an introducer at or near the proximal insertion end of the catheter and a sleeve substantially covering the catheter. Additionally, the method can include the step of sealing an anti-reflux valve to the cylindrical attachment port within the urine collection bag to prevent reverse urine flow into the catheter.

Other objects, advantages and features of the present disclosure will become apparent from a consideration of the following specification taken in conjunction with the accompanying drawings.

### Brief Description of the Drawings

Fig. 1 is a top plan view of a urine collection assembly including a catheter and a urine collection bag in accordance with the present disclosure;
Fig. 2 is a front elevational view of the urine collection assembly including the catheter and the urine collection bag which is illustrated in Fig. 1;
Fig. 3 is a front elevational view of the urine collection assembly which is illustrated in Fig. 2 with the urine collection bag partially unfolded;
Fig. 3 A is an enlarged detail view of a cylindrical barrel portion on a catheter within a cylindrical attachment port in the urine collection bag;
Fig. 3B is an enlarged detail view of the cylindrical portion of a tapered funnel on the catheter remaining after cutting off a conical portion;
Fig. 4 is a front elevational view of a catheter having a conventional tapered funnel which includes a cylindrical portion and a conical portion;
Fig. 5 is a top plan view of a urine collection bag for use with a catheter in the urine collection assembly which is illustrated in Fig. 1;
Fig. 5A is an enlarged detail view of the portion of the urine collection bag of Fig. 5 in which a cylindrical attachment port is located;
Fig. 6 is a perspective view illustrating a urine collection assembly after it is removed from a package and is in a ready-to-use condition;
Figs. 7A-7E illustrate the series of steps in folding the urine collection bag before a final folding step into the configuration illustrated in Figs. 1 and 2; and
Figs. 8A-8G illustrate an apparatus for performing a method of manufacturing a urine collection assembly in accordance with the present disclosure.

### Detailed Description of the Disclosure

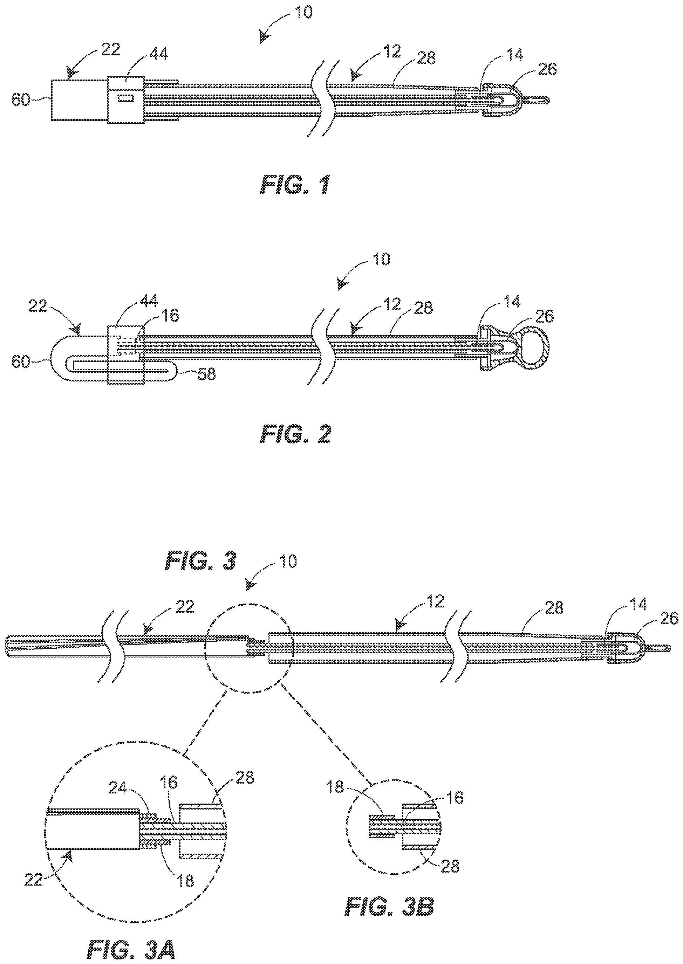

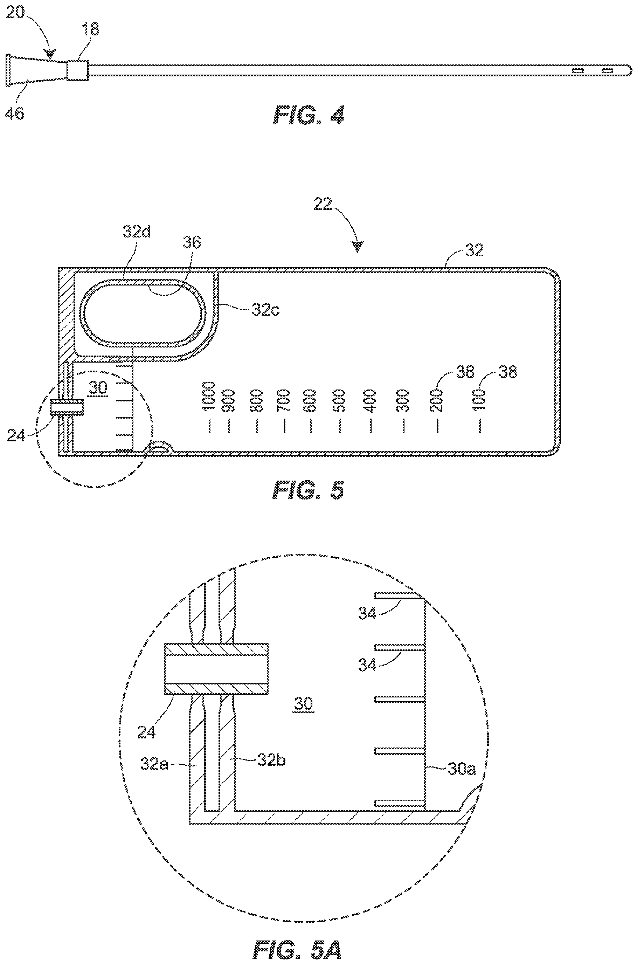

In the illustrations given, and with reference first to Figs. 1 and 2, the reference numeral 10 designates generally a urine collection assembly in accordance with the present disclosure. The urine collection assembly 10 includes a catheter generally designated 12 having a proximal insertion end 14 and a distal end 16. The distal end 16 has only a cylindrical barrel portion 18 of a tapered funnel such as 20 (Fig. 4) integrally associated therewith. The cylindrical barrel portion 18 of the tapered funnel 20 at the distal end 16 of the catheter 12 is best illustrated in the detailed views of Figs. 3A and 3B. The urine collection assembly 10 also includes a urine collection bag generally designated 22 having a cylindrical attachment port 24. The cylindrical barrel portion 18 is sealed within the cylindrical attachment port 24 (Fig. 3) to provide a closed collection system. Accordingly, the urine collection assembly 10 permits urine drained from the bladder by the catheter 12 to pass through the cylindrical barrel portion 18 and into the urine collection bag 22.

The urine collection bag 22 can be formed of flexible sheet material with the attachment port 24 sealed into the sheet material. The flexible sheet material can comprise two sheets of EVA/polyethylene film sealed about the peripheral edges thereof. The cylindrical attachment port 24 can be a co-extruded tube formed to have an EVA outer surface and a PVC inner surface. Alternatively, it will be understood that other suitable materials now known or hereafter discovered may be suitable for use.

It is advantageous to utilize these materials for the flexible sheet material and the cylindrical attachment port 24. By utilizing EVA/polyethylene film, the flexible sheet material of the urine collection bag 22 is easy to seal substantially entirely about the peripheral edges thereof, and the cylindrical barrel portion 18 may suitably comprise a portion of a tapered funnel such as 20 which is conventionally formed of PVC. As a result, the cylindrical barrel portion 18 and inner surface of the cylindrical attachment port 24 may be formed of PVC. Since these like materials are utilized and will be in confronting relation when the cylindrical barrel portion 18 is inserted into cylindrical attachment port 24 during manufacture of the urine collection assembly 10, it is possible to utilize spin welding to seal the cylindrical barrel portion 18 within the cylindrical attachment port 24 to these components together while eliminating the need for the use of adhesives or solvents that can produce harmful vapors.

As will be appreciated, the catheter 12 is disposed externally of the urine collection bag 22 in a ready-to-use position after the cylindrical barrel portion 18 has been sealed within the cylindrical attachment port 24. The catheter 12 may advantageously include a hydrated hydrophilic outer surface to further enhance the desirability of the urine collection assembly 10 by ensuring it is ready-to use without any preliminary preparation. Further, the catheter 12 may include an introducer 26 at or near the proximal insertion end 14 of the catheter 12, and it may also include a sleeve 28 substantially covering the catheter 12 to facilitate no-touch handling. The sleeve 28 may be made from polyurethane or other suitable materials now known or hereafter discovered.

Referring to Figs. 5 and 5 A, the urine collection bag 22 can include an anti -reflux valve 30 sealed to the cylindrical attachment port 24. Fig. 5 illustrates the peripheral seal 32 extending entirely about the periphery of two sheets of flexible material to form the urine collection bag 22. Also, it will be noted that the urine collection bag 22 may include two seal portions 32a and 32b in the region of the cylindrical attachment port 24. With this arrangement, it will be understood that the anti-reflux valve 30 can also be captured by one or both of the seal portions 32a and 32b.

Further, and still referring to Fig. 5, the anti-reflux valve 30 may be formed of two flexible sheets of material advantageously comprising LDPE film. When utilizing flexible sheets or films of this type, it may be advantageous to tack or weld the two sheets or films together at a plurality of discrete locations such as 34. In other words, the tacks or welds may be provided at locations opposite the cylindrical attachment port 24 in substantially equally spaced relation. With the arrangement illustrated in Figs. 5 and 5A, the discrete tacks or welds 34 are preferably evenly spaced along the edge 30a of the anti-reflux valve 30 opposite the cylindrical attachment port 24 and extend longitudinally in generally parallel relation to the axis of the cylindrical attachment port 24 to facilitate urine flow into the urine collection bag 22 but prevent reverse urine flow out of the urine collection bag 22, i.e., they form a nonreturn valve.

Fig. 5 also illustrates two further seals 32c and 32d which define an opening 36 to facilitate handling of the urine collection bag 22. In addition, the urine collection bag 22 may be provided with volume indicia as at 38 indicative of the amount of urine collected therein.

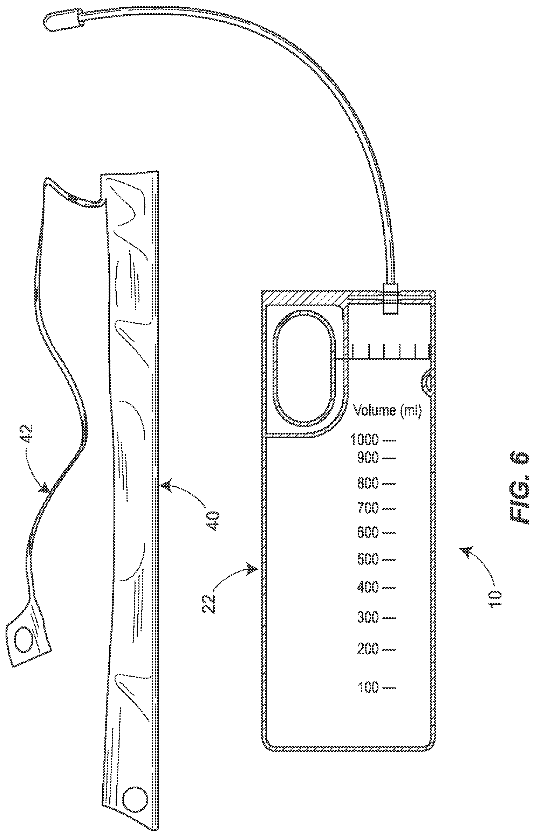

Referring to Fig. 6, it will be appreciated that the urine collection assembly 10 can be provided as a packaged, ready-to-use product. It can then include a gas impermeable package 40, illustrated after opening utilizing a tear strip 42. The catheter 12 and urine collection bag 22 are illustrated after they have been removed from the package 40 and are in a ready-to-use condition.

As illustrated in Figs. 5 and 6, the urine collection bag 22 is preferably generally rectangular in shape. This permits it to easily be folded at least once generally perpendicular to the direction of the catheter 12 and at least once in the direction of the catheter 12 so that it can be rendered quite compact after folding as illustrated in Figs. 1 and 2. When the urine collection bag 22 has been folded, a band 44 can be used to hold it in a folded condition.

By folding the urine collection bag 22 in this manner, and utilizing a band 44 to hold it in the folded condition, the urine collection assembly 10 can assume a long, narrow profile suitable for utilizing a long, narrow and compact package such as 40.

The invention is directed to a method of manufacturing a packaged ready-to-use urine collection assembly 10. The method comprises the step of providing a catheter 12 having a proximal insertion end 14 and a distal end 16 wherein the catheter 12 can have a hydrophilic outer surface to be hydrated prior to use. The distal end 16 of the catheter 12 is provided with a tapered funnel 20 (Fig. 4) including a cylindrical barrel portion 18 and a conical portion 46 integrally associated therewith. The method also comprises the step of cutting off the conical portion 46 of the tapered funnel 20 and leaving only the cylindrical barrel portion 18 of the tapered funnel 20 on the catheter 12. The method further comprises the step of providing a urine collection bag 22 having a cylindrical attachment port 24 with an opening to receive the cylindrical barrel portion 18 in size for size fashion. It will be understood that due to tolerances there may be some slight size variation between the inside diameter of the cylindrical attachment port 24 and the outside diameter of the cylindrical barrel portion 18. The cylindrical barrel portion 18 of the tapered funnel 20 is placed in the opening defined by the cylindrical attachment port 24 of the urine collection bag 22 in size for size fashion. The method additionally comprises the step of sealing the cylindrical barrel portion 18 of the tapered funnel 20 within the opening defined by the cylindrical attachment port 24 of the urine collection bag 22.

In addition to the foregoing, the method according to the invention includes the step of providing a gas impermeable package 40 capable of receiving the catheter 12 and the urine collection bag 22 therein, the step of placing the catheter 12, the urine collection bag 22 and a hydrating agent into the gas impermeable package 40, and the step of sealing the gas impermeable package 40 with the catheter 12, the urine collection bag 22 and the hydrating agent therein to complete the ready-to-use assembly.

The method can include the steps of forming the urine collection bag 22 of a flexible sheet material and sealing the attachment port 24 into the sheet material. It can also include the step of using two sheets of EVA/polyethylene film for the sheet material and sealing the two sheets about the peripheral edges 32. It can also include the step of providing a co- extruded tube for the cylindrical attachment port 24 formed with an EVA outer surface and PVC inner surface.

Preferably, the cylindrical barrel portion 18 of the tapered funnel 20 is formed of PVC and the cylindrical barrel portion 18 is sealed within the opening defined by the cylindrical attachment port 24 by spin welding. Still additionally, the spin welding step is advantageously performed by holding the catheter 12 in a fixed or stationary position and then causing the urine collection bag 22 to spin relative to the stationary catheter 12.

The spin welding step can include spinning the urine collection bag 22 relative to the catheter 12 at a rotational speed of between 1900 and 3000 RPMs, depending upon the size of the catheter, and spinning the urine collection bag 22 relative to the catheter 12 for between 14 and 16 revolutions, again depending upon the size of the catheter. The step of providing a urine collection bag 22 may include providing it in a generally rectangular shape, folding the urine collection bag 22 at least once in the direction of the catheter 12, folding the urine collection bag 22 at least once generally perpendicular to the direction of the catheter 12, performing the spin welding step, again folding the urine collection bag 22 in the direction of the catheter 12, and banding the urine collection bag 22 with a band 44 in a folded condition for placement a package such as 40.

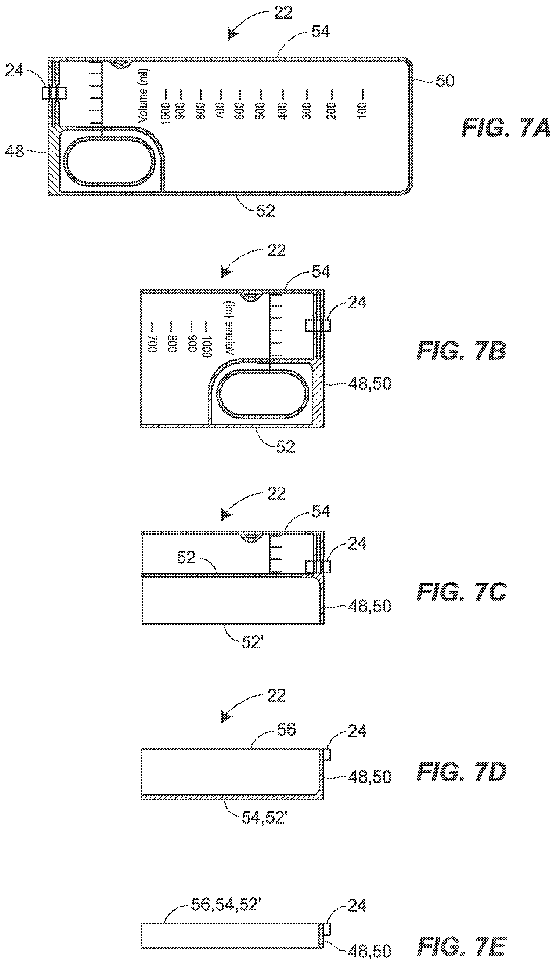

In this connection, Figs. 7A-7e illustrate a urine collection bag folding process in accordance with the disclosure. The urine collection bag 22 is laid flat in order to initiate the folding process (Fig. 7A), and the urine collection bag 22 is folded in half in the direction of the cylindrical attachment port 24 (and, thus, in the direction of the catheter) so the left edge 48 containing the cylindrical attachment port 24 meets the right edge 50 (Fig. 7B). Next, the bottom edge 52 of the urine collection bag 22 is folded up in a direction generally perpendicular to the cylindrical attachment port 24 (and, thus, generally perpendicular to the direction of the catheter) to touch the bottom of the cylindrical attachment port 24 and define a new bottom edge 52' after folding (Fig. 7C). Next, the top edge 54 of the urine collection bag 22 is folded down in a direction generally perpendicular to the cylindrical attachment port 24 (and, thus, generally perpendicular to the direction of the catheter) to meet the bottom edge 52' and define a new top edge 56 after folding (Fig. 7D). Finally, the edges 54, 52' which meet at the bottom of the urine collection bag 22 in Fig. 7D are folded up in a direction generally perpendicular to the cylindrical attachment port 24 (and, thus, generally perpendicular to the direction of the catheter) to meet the top edge 56 after which the urine collection bag is ready for spin welding.

After the cylindrical barrel portion 18 of a tapered funnel 20 on a catheter 12 has been sealed to the inner surface of the cylindrical attachment port 24 by spin welding (Fig. 3), the urine collection bag can be folded in the direction of the catheter 12 as at 58 and 60 (Figs. 1 and 2) and banded with a band 44 in the folded condition shown for placement in a package such as 40 (Fig. 6).

Further aspects of the method can include the steps of providing an introducer 26 at or near the proximal insertion end 14 of the catheter 12 and a sleeve 28 substantially covering the catheter 12. The method can also include the step of sealing an anti-reflux valve 30 at a location within the urine collection bag 22 to the cylindrical attachment port 24 and/or sealing the anti-reflux valve 30 to the urine collection bag 22 such as by the seals 32a and 32b. Still further, the method can include the step of providing liquid water as the hydrating agent in the package 40 and including isolating the liquid water from the hydrophilic outer surface of the catheter.

When isolated liquid water is used as the hydrating agent, the method still additionally includes the step of delaying distribution of the gas impermeable package 40 before use of the catheter 12 for a period of time sufficient: i) to form a 100% relative humidity atmosphere within the sealed gas impermeable package 40, and ii) for the hydrophilic outer surface of the catheter 12 to be hydrated so the catheter 12 is ready for use.

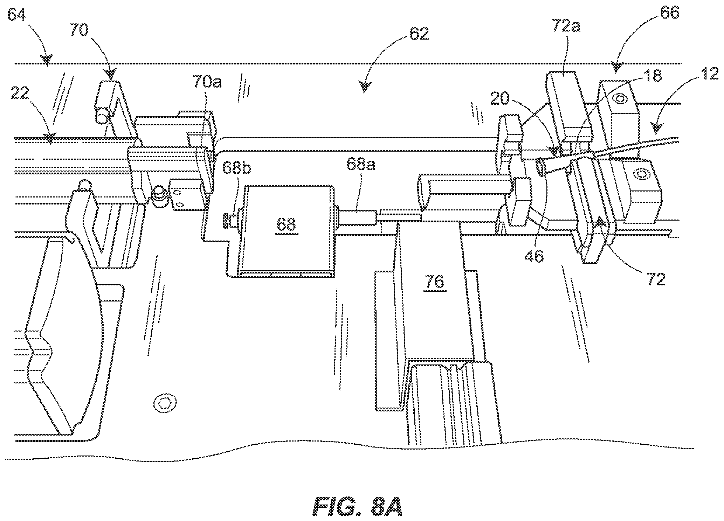

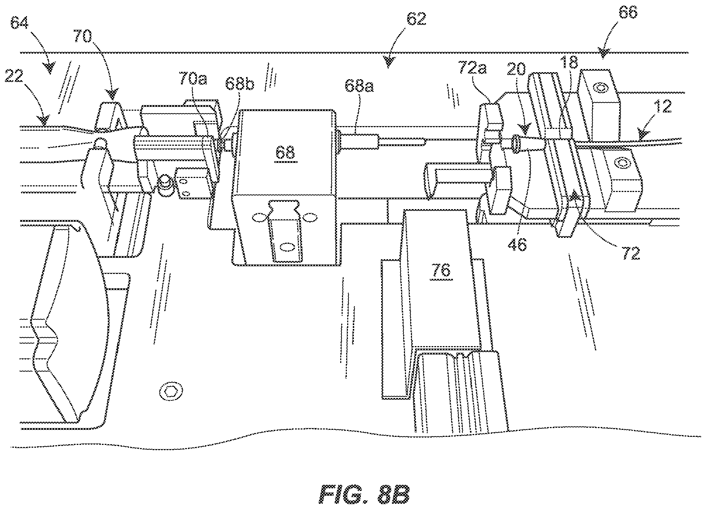

Referring to Figs. 8A-8G, the reference numeral 62 designates generally an apparatus for performing certain steps of the method of manufacturing a urine collection assembly 10 in accordance with the present disclosure. The apparatus 62 includes a receiving station 64 for the urine collection bag 22 and a receiving station 66 for the conventional catheter 12 wherein the urine collection bag 22 and the catheter 12 are placed in the respective receiving stations 64 and 66 as illustrated in Fig. 8A. After the urine collection bag 22 and the catheter 12 have been placed in the respective receiving stations 64 and 66, a station 68 which supports a mandrel 68a for the funnel 20 on the catheter 12 and which supports a positioning element 68b for the cylindrical attachment port 24 on the urine collection bag 22 is raised as shown in Fig. 8B. It will also be noted that a urine collection bag gripping element 70 is provided for gripping the urine collection bag 22 and a funnel gripping element 72 is provided for gripping the funnel 20 on the catheter 12 (Fig. 8B). By comparing Figs. 8A and 8B, it can be seen that the gripping elements 70 and 72 undergo linear reciprocating movement between non-gripping positions (Fig. 8A) and positions in which they grip the urine collection bag 22 and the funnel 20 on the catheter 12, respectively (Fig. 8B). In addition, it should be noted that the receiving station 66 and the gripping element 70 are each mounted for movement toward and away from the station 68 for reasons that will become clear in the description which follows.

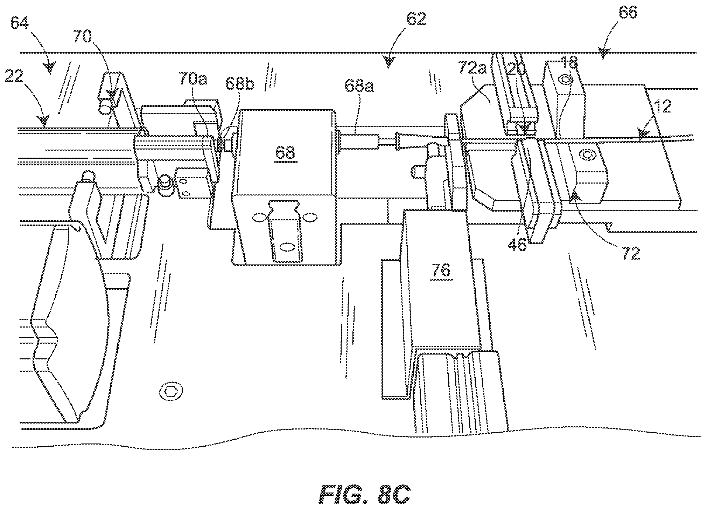

Referring to Fig. 8C, the apparatus 62 is shown in a position after the gripping element 70 has moved the urine collection bag 22 toward the station 68 to a location where the positioning element 68b properly positions the cylindrical attachment port 24. It is also shown in a position after the receiving station 66 has moved the catheter 12 to properly position the funnel 20 on the mandrel 68a. At this point, the funnel gripping element 72 releases the funnel 20, the station 66 moves away from the station 68, and a second funnel gripping element 72a moves to grip the cylindrical barrel portion 18 of the funnel 20. Further, it will be noted that a cutting element 74 has been moved linearly from within a guard 76 to a position where it can cut off the conical portion 46 leaving only the cylindrical barrel portion 18 of the funnel 18 (see, also, Fig. 4).

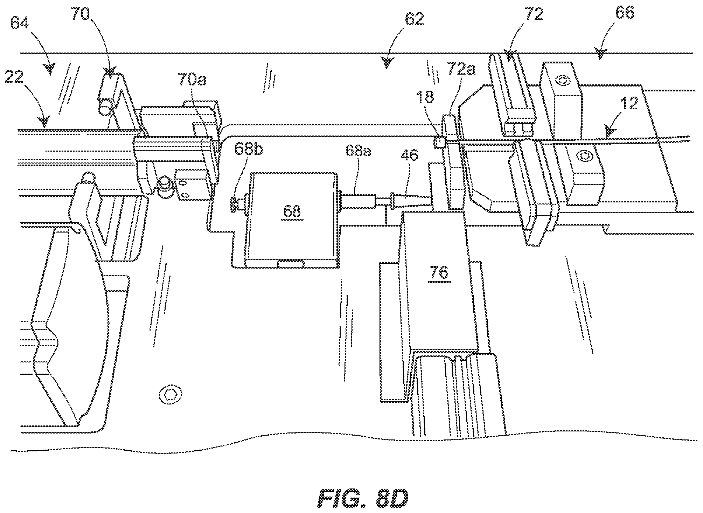

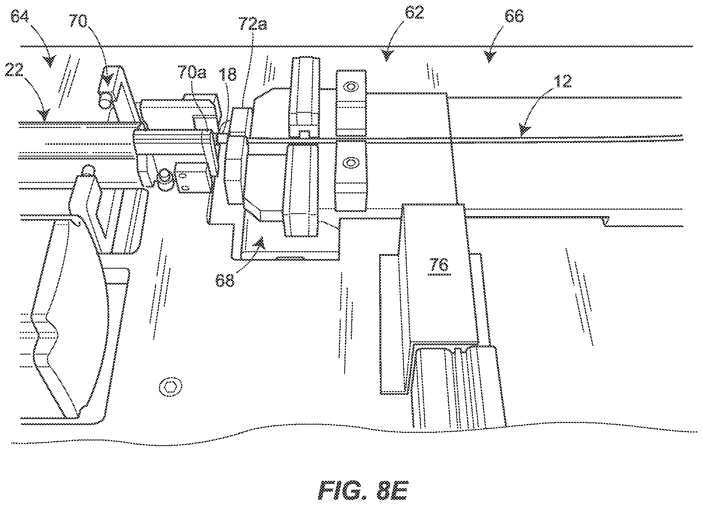

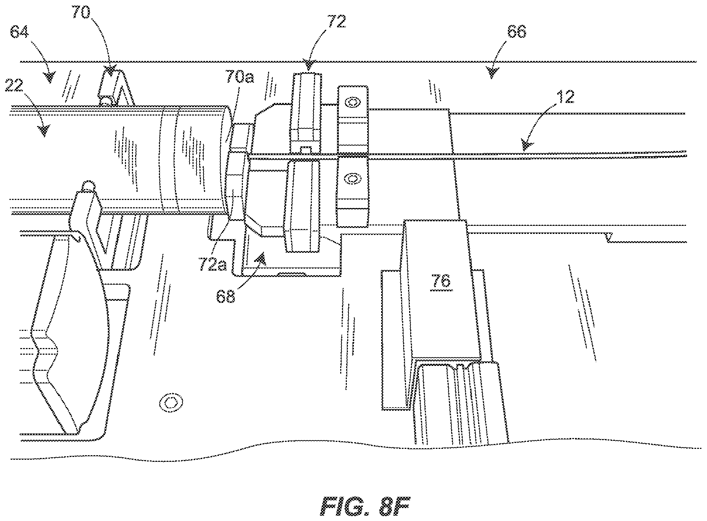

After the conical portion 46 of the funnel 20 has been cut off, the cutting element 74 is withdrawn into the guard 76 and the station 68 is dropped down (as shown in Fig. 8D) for removal of the severed conical portion 46 of the funnel 20 from the mandrel 68a. Next, the station 66 is moved toward the station 64 (as shown in Fig. 8E) until the cylindrical barrel portion 18 of the funnel 20 is disposed within the cylindrical attachment port 24 in size to size fashion. Finally, the second funnel gripping element 72a grips the cylindrical barrel portion 18 of the funnel 20 to maintain it in a stationary position and a second urine collection bag gripping element 70a grips and spins both the cylindrical attachment port 24 and the urine collection bag 22 relative to the catheter 12 (Fig. 8F). More specifically, the second urine collection bag gripping element 70a grips and initiates the spinning of the cylindrical attachment port 24 and the urine collection bag 22 just before the cylindrical barrel portion 18 is inserted into the cylindrical attachment port 24 and, then, it accelerates to full speed after the cylindrical barrel portion 18 has been fully inserted.

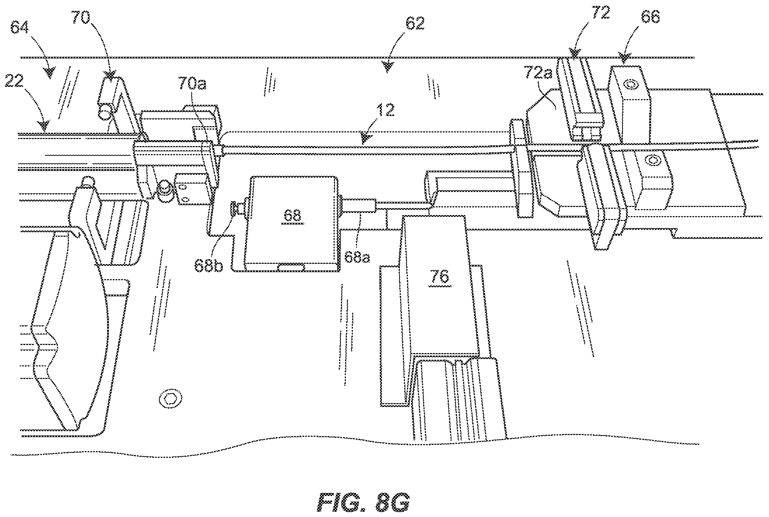

Referring to Fig. 8G, the apparatus 62 is shown after spinning which completes the manufacture of the urine collection assembly 10. The stations 64 and 66 are again in their starting positions to repeat the process. The cylindrical barrel portion 18 is bonded to the cylindrical attachment port 24 by spin welding without the use of any adhesives or solvents.

While the foregoing sets forth a detailed description of the urine collection assembly and method of the present disclosure, it will be appreciated that the scope of protection is only to be limited by the appended claims.

## Claims

1. A method of manufacturing a packaged ready-to-use urine collection assembly (10), comprising the steps of:
providing a catheter (12) having a proximal insertion end (14) and a distal end (16) having a tapered funnel (20) including a cylindrical barrel portion (18) and a conical portion (46) integrally associated therewith, the catheter also having a hydrophilic outer surface to be hydrated prior to using the catheter;
cutting off the conical portion (46) of the tapered funnel and leaving only the cylindrical barrel portion (18) of the tapered funnel integrally associated with the distal end of the catheter;
providing a urine collection bag (22) having a cylindrical attachment port (24) defining an opening sized to receive the cylindrical barrel portion (18) of the tapered funnel in size for size fashion;
placing the cylindrical barrel portion (18) of the tapered funnel into the opening defined by the cylindrical attachment port of the urine collection bag in size for size fashion;
sealing the cylindrical barrel portion of the tapered funnel within the opening defined by the cylindrical attachment port of the urine collection bag;
providing a gas impermeable package (40) capable of receiving the catheter and the urine collection bag therein;
placing the catheter, the urine collection bag and a hydrating agent into the gas impermeable package (40); and
sealing the gas impermeable package (40) with the catheter (12), the urine collection bag (22) and the hydrating agent therein.

2. The method of claim 1 including the steps of forming the urine collection bag (22) of a flexible sheet material and sealing the attachment port into the sheet material.

3. The method of claim 2 including the step of using two sheets of EVA /polyethylene film for the flexible sheet material and sealing the two sheets about the peripheral edges (32) thereof.

4. The method of claim 2 providing a co-extruded tube formed to have an EVA outer surface and a PVC inner surface for the cylindrical attachment port (24).

5. The method of claim 4 wherein the cylindrical barrel portion is formed of PVC and the step of sealing the cylindrical barrel portion within the opening is performed by spin welding.

6. The method of claim 5 wherein the spin welding step is performed by holding the catheter (12) in a stationary position and spinning the urine collection bag (22) relative to the catheter.

7. The method of claim 6 including the step of providing the urine collection bag (22) in a generally rectangular shape, folding the urine collection bag at least once generally perpendicular to the direction of the catheter (12), performing the spin welding step, folding the urine collection bag at least once in the direction of the catheter, and banding the urine collection bag in a folded condition.

8. The method of claim 7 wherein the spin welding step includes spinning the urine collection bag (22) relative to the catheter (12) at a rotational speed of between 1900 and 3000 RPMs.

9. The method of claim 8 wherein the spin welding step includes spinning the urine collection bag (22) relative to the catheter (12) for between 14 and 16 revolutions.

10. The method of claim 1 including the step of providing an introducer (26) at or near the proximal insertion end (14) of the catheter and a sleeve (28) substantially covering the catheter.

11. The method of claim 1 including the step of sealing an anti-reflux valve (30) to the cylindrical attachment port (24) within the urine collection bag.

12. The method of claim 1 wherein the hydrating agent is liquid water and including the step of isolating the liquid water from the hydrophilic outer surface of the catheter.

13. The method of claim 12 including the step of delaying distribution of the gas impermeable package (40) for use of the catheter for a period of time sufficient to form a 100% relative humidity atmosphere within the sealed gas impermeable package and for the hydrophilic outer surface of the catheter to be hydrated so the catheter is ready for use.

## Patentansprüche

1. Verfahren zur Herstellung einer verpackten, gebrauchsfertigen Urinsammelanordnung (10), das die folgenden Schritte umfasst:
Bereitstellen eines Katheters (12), der ein proximales Einschubende (14) und ein distales Ende (16) mit einem sich verjüngenden Trichter (20) aufweist, der einen zylindrischen Schaftabschnitt (18) und einen einstückig damit verbundenen konischen Abschnitt (46) beinhaltet, wobei der Katheter zudem eine hydrophile Außenfläche aufweist, die vor der Verwendung des Katheters zu hydratisieren ist;
Abschneiden des konischen Abschnitts (46) des sich verjüngenden Trichters und Belassen nur des zylindrischen Schaftabschnitts (18) des sich verjüngenden Trichters, der einstückig mit dem distalen Ende des Katheters verbunden ist;
Bereitstellen eines Urinsammelbeutels (22) mit einem zylindrischen Befestigungsanschluss (24), der eine Öffnung definiert, die so bemessen ist, dass sie den zylindrischen Schaftabschnitt (18) des sich verjüngenden Trichters in einer Weise je nach Größe aufnimmt;
Anordnen des zylindrischen Schaftabschnitts (18) des sich verjüngenden Trichters in der Öffnung, die durch den zylindrischen Befestigungsanschluss des Urinsammelbeutels definiert ist, in einer Weise je nach Größe;
Abdichten des zylindrischen Schaftabschnitts des sich verjüngenden Trichters innerhalb der Öffnung, die durch den zylindrischen Befestigungsanschluss des Urinsammelbeutels definiert ist;
Bereitstellen einer gasundurchlässigen Verpackung (40), die den Katheter und den Urinsammelbeutel darin aufnehmen kann;
Anordnen des Katheters, des Urinsammelbeutels und eines Hydratisierungsmittels in der gasundurchlässigen Verpackung (40); und
Abdichten der gasundurchlässigen Verpackung (40) mit dem Katheter (12), dem Urinsammelbeutel (22) und dem Hydratisierungsmittel darin.

2. Verfahren nach Anspruch 1, beinhaltend die Schritte des Bildens des Urinsammelbeutels (22) aus einem flexiblen Schichtmaterial und des Abdichtens des Befestigungsanschlusse in dem Schichtmaterial.

3. Verfahren nach Anspruch 2, beinhaltend den Schritt des Verwendens von zwei Schichten aus EVA-/Polyethylenfolie für das flexible Schichtmaterial und Abdichten der zwei Schichten um deren Umfangskanten (32).

4. Verfahren nach Anspruch 2, das einen koextrudierten Schlauch bereitstellt, der so gebildet ist, dass er eine EVA-Außenfläche und eine PVC-Innenfläche für den zylindrische Befestigungsanschluss (24) aufweist.

5. Verfahren nach Anspruch 4, wobei der zylindrische Schaftabschnitt aus PVC gebildet ist und der Schritt des Abdichtens des zylindrischen Schaftabschnitts innerhalb der Öffnung durch Rotationsschweißen durchgeführt wird.

6. Verfahren nach Anspruch 5, wobei der Rotationsschweißschritt durchgeführt wird, indem der Katheter (12) in einer stationären Position gehalten wird und der Urinsammelbeutel (22) relativ zu dem Katheter gedreht wird.

7. Verfahren nach Anspruch 6, beinhaltend den Schritt des Bereitstellens des Urinsammelbeutels (22) in einer allgemein rechteckigen Form, des Faltens des Urinsammelbeutels mindestens einmal allgemein senkrecht zur Richtung des Katheters (12), des Durchführens des Rotationsschweißschritts, des Faltens des Urinsammelbeutels mindestens einmal in Richtung des Katheters und des Banderolierens des Urinsammelbeutels in einem gefalteten Zustand.

8. Verfahren nach Anspruch 7, wobei der Rotationsschweißschritt das Drehen des Urinsammelbeutels (22) relativ zu dem Katheter (12) mit einer Drehgeschwindigkeit zwischen 1900 und 3000 U/min beinhaltet.

9. Verfahren nach Anspruch 8, wobei der Rotationsschweißschritt das Drehen des Urinsammelbeutels (22) relativ zu dem Katheter (12) für 14 bis 16 Umdrehungen beinhaltet.

10. Verfahren nach Anspruch 1, beinhaltend den Schritt des Bereitstellens einer Einführhilfe (26) an oder nahe dem proximalen Einschubende (14) des Katheters und einer Hülse (28), die den Katheter im Wesentlichen bedeckt.

11. Verfahren nach Anspruch 1, beinhaltend den Schritt des Abdichtens eines Antirückflussventils (30) an dem zylindrischen Befestigungsanschluss (24) innerhalb des Urinsammelbeutels.

12. Verfahren nach Anspruch 1, wobei das Hydratisierungsmittel flüssiges Wasser ist, und beinhaltend den Schritt des Isolierens des flüssigen Wassers von der hydrophilen Außenfläche des Katheters.

13. Verfahren nach Anspruch 12, beinhaltend den Schritt des Verzögerns der Ausgabe der gasundurchlässigen Verpackung (40) zur Verwendung des Katheters für eine Zeitspanne, die ausreicht, um eine Atmosphäre mit 100 % relativer Feuchtigkeit innerhalb der abgedichteten gasundurchlässigen Verpackung zu bilden und die hydrophile Außenfläche zu hydratisieren, sodass der Kathether gebrauchsfertig ist.

## Revendications

1. Procédé de fabrication d'un ensemble de collecte d'urine prêt à l'emploi conditionné (10), comprenant les étapes de :
fourniture d'un cathéter (12) ayant une extrémité d'insertion proximale (14) et une extrémité distale (16) ayant un entonnoir conique (20) comportant une partie de corps cylindrique (18) et une partie conique (46) associée intégralement à celle-ci, le cathéter ayant également une surface externe hydrophile à hydrater avant d'utiliser le cathéter ;
découpe de la partie conique (46) de l'entonnoir conique et ne laisser que la partie de corps cylindrique (18) de l'entonnoir conique intégralement associée à l'extrémité distale du cathéter ;
fourniture d'une poche de collecte d'urine (22) ayant un orifice de fixation cylindrique (24) définissant une ouverture dimensionnée pour recevoir la partie de corps cylindrique (18) de l'entonnoir conique avec une correspondance de dimensions ;
placement de la partie de corps cylindrique (18) de l'entonnoir conique dans l'ouverture définie par l'orifice de fixation cylindrique de la poche de collecte d'urine avec une correspondance de dimensions ;
scellement de la partie de corps cylindrique de l'entonnoir conique à l'intérieur de l'ouverture définie par l'orifice de fixation cylindrique de la poche de collecte d'urine ;
fourniture d'un emballage imperméable aux gaz (40) capable de recevoir le cathéter et la poche de collecte d'urine à l'intérieur ;
placement du cathéter, de la poche de collecte d'urine et d'un agent hydratant dans l'emballage imperméable aux gaz (40) ; et
scellement de l'emballage imperméable aux gaz (40) avec le cathéter (12), de la poche de collecte d'urine (22) et de l'agent hydratant à l'intérieur.

2. Procédé selon la revendication 1, comportant les étapes de formage de la poche de collecte d'urine (22) en un matériau en feuille flexible et de scellement de l'orifice de fixation dans le matériau en feuille.

3. Procédé selon la revendication 2 comportant l'étape d'utilisation de deux feuilles d'EVA / de film polyéthylène pour le matériau en feuille flexible et scellement des deux feuilles autour des bords périphériques (32) de celles-ci.

4. Procédé selon la revendication 2, fournissant un tube coextrudé formé de manière à avoir une surface externe en EVA et une surface interne en PVC pour l'orifice de fixation cylindrique (24).

5. Procédé selon la revendication 4, dans lequel la partie de corps cylindrique est constituée de PVC et l'étape de scellement de la partie de corps cylindrique à l'intérieur de l'ouverture est réalisée par soudage par rotation.

6. Procédé selon la revendication 5, dans lequel l'étape de soudage par rotation est réalisée en maintenant le cathéter (12) dans une position stationnaire et en faisant tourner la poche de collecte d'urine (22) par rapport au cathéter.

7. Procédé selon la revendication 6, comportant l'étape de fourniture de la poche de collecte d'urine (22) sous une forme généralement rectangulaire, de pliage de la poche de collecte d'urine au moins une fois généralement perpendiculairement à la direction du cathéter (12), de réalisation de l'étape de soudage par rotation, de pliage de la poche de collecte d'urine au moins une fois dans la direction du cathéter, et de cerclage de la poche de collecte d'urine dans un état plié.

8. Procédé selon la revendication 7, dans lequel l'étape de soudage par rotation comporte la rotation de la poche de collecte d'urine (22) par rapport au cathéter (12) à une vitesse de rotation comprise entre 1900 et 3000 tr/min.

9. Procédé selon la revendication 8, dans lequel l'étape de soudage par rotation comporte la rotation de la poche de collecte d'urine (22) par rapport au cathéter (12) pendant 14 à 16 révolutions.

10. Procédé selon la revendication 1, comportant l'étape de fourniture d'un introducteur (26) au niveau ou à proximité de l'extrémité d'insertion proximale (14) du cathéter et d'un manchon (28) recouvrant sensiblement le cathéter.

11. Procédé selon la revendication 1, comportant l'étape de scellement d'une valve anti-reflux (30) à l'orifice de fixation cylindrique (24) à l'intérieur de la poche de collecte d'urine.

12. Procédé selon la revendication 1, dans lequel l'agent hydratant est de l'eau liquide et comportant l'étape d'isolement de l'eau liquide de la surface externe hydrophile du cathéter.

13. Procédé selon la revendication 12, comportant l'étape de retardement de la distribution de l'emballage imperméable aux gaz (40) pour l'utilisation du cathéter pendant une période de temps suffisante pour former une atmosphère d'humidité relative de 100 % à l'intérieur de l'emballage imperméable aux gaz scellé et pour la surface externe hydrophile du cathéter à hydrater afin que le cathéter soit prêt à l'emploi.
